# EUROPÄISCHE PATENTANMELDUNG

(11) **EP 0 911 387 A2**
(43) Veröffentlichungstag der Anmeldung: **28.04.1999**
(21) Anmeldenummer: 98119803.9
(22) Anmeldetag: 19.10.1998
(51) Int. Cl.: C12N 1/14, A01N 63/04, B09C 1/10

(54) **Rekultivierung von durch Schwermetalle, Radionuklide, Herbizide oder Salze verseuchten Böden**

(30) Priorität: 20.10.1997 DE 19746300
(71) Anmelder: Bothe, Hermann Prof. Dr., 50374 Erfstadt (DE)
(72) Erfinder: Bothe, Hermann Prof. Dr., 50374 Erfstadt (DE); Kaldorf, Michael Dr., 41189 Mönchengladbach (DE); Hildebrandt, Ulrich, 41189 Mönchengladbach (DE)
(74) Vertreter: Vossius, Volker, Dr.

(57) **Zusammenfassung**

Die Erfindung liegt auf dem Gebiet der Rekultivierung von insbesondere durch Schwermetalle, Radionuklide, Herbizide und/oder Salze verseuchten Böden mit Hilfe von Pilzen der Gattung *Glomus* oder deren Sporen.

## Beschreibung

Die Erfindung liegt auf dem Gebiet der Rekultivierung von insbesondere durch Schwermetalle, Radionuklide, Herbizide und/oder Salze verseuchten Böden.

Weltweit existieren verseuchte Landschaften, die derzeit nicht in Kultur genommen werden können, da die Böden eine hohe Konzentration von z.B. Schwermetallen, Radionukliden, Herbiziden oder Salzen enthalten, die das Wachstum von Nutzpflanzen hemmen. Die Ansammlung solcher Substanzen oder Elemente im Boden hemmt oder unterdrückt das natürliche Wachstum von Pflanzen wie natürlicher Flora, Zier- oder Nutzpflanzen, so daß die Landschaften brach liegen.

Im Laufe der Evolution haben Pflanzen eine mehr oder minder starke Toleranz gegenüber z.B. Schwermetallen entwickelt. Als Schwermetalle werden jene Elemente des Periodensystems bezeichnet, die eine Dichte von über 5,0g/cm 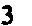 aufweisen. Tische Beispiele sind Zink (Zn), Blei (Pb), Quecksilber (Hg) und Cadmium (Cd). Gegen Schwermetalle tolerante Pflanzenarten bzw. Ökotypen werden auch Metallophyten genannt (Ernst, 1982). Dazu zählen Pflanzen wie *Viola calaminaria* und *Viola guestphalica* (vormals *Viola calaminaria* ssp. *westfalica*), die ausschließlich auf schwermetallhaltigen Böden vorkommen und als absolute Metallophyten bezeichnet werden. Auch viele andere Arten wie *Armeria maritima* ssp. *calaminaria*, *Thlaspi alpestre* ssp. *calaminare, Minuartia verna* und *Silene cucubalus* var. *humilis* gelten als Metallophyten oder Schwermetallpflanzen. Metallophyten, die ein Schwermetall mit über 1000µg/g Trockengewicht anreichern, werden als Hyperakkumulatoren bezeichnet. Dazu zählen z.B. *Thlaspi alpestre* und *Minuartia verna* für Zink sowie *Armeria maritima* für Zink und Blei (Schlee, 1992). *Viola guestphalica* besitzt weltweit nur einen einzigen natürlichen Standort bei Blankenrode im Kreis Paderborn (Westfalen) und stellt somit einen spektakulären Fall von Lokalendemismus dar. *Viola calaminaria* kommt ausschließlich auf schwermetallhaltigen Böden im Aachener Raum und in Ostbelgien vor. Aufgrund des äußerst lokal begrenzten Vorkommens dieser beiden Metallophyten sind diese Pflanzen bisher nur wenig beachtet und untersucht worden.

Toleranz gegen Schwermetalle beruht auf unterschiedlichen Mechanismen: Den Ausschluß, die Bindung an die Zellwand, die Komplexbildung durch pflanzliche Chelatbildner, die Komplexbildung mit organischen Säuren, den Transport und die Verteilung im Sinne einer Verdünnung (Harborne, 1995). Schwermetalle kommen im Wurzelgewebe vornehmlich in austauschbarer, in den Blättern jedoch in wasserlöslicher Form vor. In den Wurzeln erfolgt dabei vorwiegend eine Bindung an die Zellwand, in den Blättern hingegen eine Komplexierung in den Vakuolen. Beim Transport vom Cytoplasma in die Vakuole können die Schwermetalle ihre toxische Wirkung entfalten (Ernst, 1972). Die Toleranz ist metallspezifisch, apgepaßt an die Bodenverhältnisse des natürlichen Standortes. Pflanzen zinkreicher Böden sind nur gegen Zink, Pflanzen kupferreicher Böden gegen Kupfer, aber Pflanzen kupfer- und zinkreicher Böden gegen beide Schwermetalle tolerant (Ernst, 1972). Die Schwermetalltoleranz ist genotypisch festgelegt, d.h. vererbbar (Ernst, 1982). Die genetischen Voraussetzungen sind jedoch möglicherweise auch bei nichttoleranten Ökotypen gegeben. Nach Untersuchungen von Baker und Walker (1990) können schwermetalltolerante Arten nach mehreren Generationen in unbelasteten Böden ihre Schwermetalltoleranz wieder einbüßen.

Auch gegenüber Ionen von in größerer Konzentration vorkommenden Salzen wie Kochsalz (NaCl) haben einige Pflanzenarten Toleranzen entwickelt. Diese Pflanzen, die in salzreichen Habitaten wie Salzmarschen, Salzwüsten etc. vorkommen, werden als Halophyten bezeichnet und sind in der Lage, auf Böden mit einem Kochsalzgehalt von bis zu 20% zu wachsen (Levitt, 1980). Typische Halophyten sind z.B. *Armeria maritima, Aster tripolium, Plantago maritima, Salicornia europaea* u.v.a.. Eine Anpassung an den Salzgehalt des Bodens kann über verschiedene Wege erreicht werden: Die Anreicherung von NaCl in den Vakuolen, Verhinderung des Eindringens von NaCl in die Zelle und die Verdünnung von NaCl nach dem Eindringen in die Pflanze (Harborne, 1995). Es existieren bei vielen Halophyten Mechanismen, die auf einer Anreicherung von nicht-toxischen Osmotika wie Prolin, Glycinbetain, Sulfonderivaten oder Polyolen im Cytoplasma beruhen, die einen Ausgleich des durch anorganische Ionen verminderten Wasserpotentials in den Vakuolen ermöglichen (Harborne, 1995).

Arbuskuläre Mykorrhizapilze kommen in mit Schwermetallen kontaminierten Böden vor (Weissenhorn et al., 1993; Griffioen, 1994) und fungieren ebenfalls als Symbionten von Pflanzen saliner Standorte (Khan, 1974; Allen und Cunningham, 1983; Pond et al., 1984; Rozema et al., 1986; Ho, 1987). Schwermetalle und erhöhte NaCl-Konzentrationen sind in der Lage, Keimung und Wachstum von AM-Pilzen zu hemmen und im Falle der Schwermetalle eine Mykorrhizierung von Pflanzen in Kulturexperimenten zu verhindern (Gildon und Tinker, 1983; Hirrel, 1981; Estaun, 1989; Juniper und Abbott, 1991). Dabei erwiesen sich aus schwermetallbelasteten Böden isolierte AM-Sporen hinsichtlich ihrer Keimungskapazität toleranter gegenüber Schwermetallen als Kontrollisolate (Gildon und Tinker, 1981; Weissenhorn et al., 1994). Der Einfluß von AM-Pilzen auf das Wachstum von Pflanzen in mit Schwermetallen belasteten Böden wurde bisher nicht eindeutig beschrieben. Killham und Firestone (1983) beobachteten bei mit AM-Pilzen der Gattung *Glomus fasciculatum* infizierten Gräsern einen negativen Effekt auf das Wachstum, der auf durch Mykorrhizierung verursachte, erhöhte Schwermetallaufnahme in die Pflanze zurückgeführt wurde. Dueck et al. (1986) beobachteten jedoch für zwei verschiedene Gräser eine Wachstumsstimulierung durch AM-Pilzinfektion der Gattung *Glomus fasciculatum* isoliert aus einem Dünengebiet bestanden mit *Festuca rubra* (kein natürlicher Schwermetallstandort, duch Industrie leicht belastet) in einem Zn-belasteten System. Weissenhorn et al. (1995b) konnten keine wesentlichen Wachstumsvorteile für mit AM-Pilzen infizierte Maispflanzen in schwermetallbelasteter Erde aufzeigen. Diese Mykorrhizapilz-Population stammte von einer landwirtschaflich genutzten Fläche, die durch eine nahegelegene Bleihütte mit Zink, Blei und Cadmium gering belastet war.

Eine Rekultivierung von Landschaften, deren Böden mit hohen Konzentration von z.B. Schwermetallen, Radionukliden, Herbiziden oder Salzen verseucht sind, ist bisher ohne eine zuvor aufwendige und kostenintensive Abtragung der belasteten Bodenschichten noch nicht gelungen. Es besteht somit der Bedarf solche Landschaften auf natürliche Weise zu rekultivieren und wirtschaftlich zu nutzen.

Der vorliegenden Erfindung liegt deshalb die Aufgabe zugrunde, arbuskuläre Mykorrizha-Pilze bereitzustellen, die zur Rekultivierung der verseuchten Landschaften, die z.B. mit Schwermetallen, Radionukliden, Herbiziden oder Salzen belastet sind, verwendet werden können.

Die Aufgabe wird erfindungsgemäß durch isolierte und gereinigte Pilze der Gattung *Glomus* und deren Sporen, die mit Metallophyten ein arbuskuläres Mykorrhizageflecht ausgebildet haben, gelöst. Gegen Schwermetalle tolerante Pflanzenarten bzw. Ökotypen werden Metallophyten genannt (Ernst, 1982). Der Ausdruck Schwermetalle bedeutet jene Elemente des Periodensystems, die eine Dichte von über 5,0g/cm 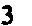 aufweisen. Typische Beispiele Zink (Zn), Blei (Pb), Quecksilber (hg) und Cadmium (Cd). Der Ausdruck Metallophyten umfaßt Arten wie *Armeria maritima* ssp. *calaminaria*, *Thlaspi alpestre* ssp. *calaminare* und *Minuartia verna*. Metallophyten, die ein Schwermetall mit über 1000µg/g Trockengewicht anreichern, werden als Hyperakkumulatoren bezeichnet (Brooks et al., 1980). Dazu zählen z.B. *Thlaspi alpestre* und *Minuartia verna* für Zink sowie *Armeria maritima* für Zink und Blei (Schlee, 1992). Bevorzugt sind Metallophyten ausgewählt aus der Gruppe *Viola calaminaria* und *Viola guestphalica* (vormals *Viola calaminaria* ssp. *westfalica*). Pflanzen, die in salzreichen Habitaten wie Salzmarschen, Salzwüsten etc. vorkommen, werden als Halophyten bezeichnet und sind in der Lage, auf Böden mit einem Salzgehalt von bis zu 20% zu wachsen (Levitt, 1980). Typische Halophyten sind z.B. *Armeria maritima, Aster tripolium, Plantago maritima, Salicornia europaea*. Beispiele für Salze sind NaCl oder KCl.

Die Metallophyten können an Standorten mit natürlicherweise hohem Schwermetallgehalt im Boden sowie an Standorten, die durch Erzabbau, Verhüttung oder andere anthropogene Einflüsse dauerhaft hohe Schwermetallgehalte im Boden aufweisen gefunden werden. Insbesondere können Metallophyten der Gattung *Viola calaminaria* im und am Rande des Naturschutzgebietes "Schlangenberg" in Stolberg-Breiningerberg und *Viola guestphalica* im und am Rande des Naturschutzgebietes 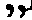 Bleikuhlen" in Lichtenberg-Blankenrode gefunden werden.

Die erfindungsgemäßen Pilze können wie folgt isoliert werden: Nicht speziell behandelte, mykorrhizierte Wurzeln von *Viola calaminaria* wurden zusammen mit steril gekeimten Maiskörnern in Töpfe mit einer Mischung aus 90% Lecaton / 10 % Einheitserde gelegt. Die aus diesen Wurzelsegmenten von *Viola* auswachsenden *Glomus*-Hyphen haben dann die Maiswurzeln infiziert. Nach 90 Tagen wird dann das Substrat einschließlich Maiswurzeln, *Glomus*-Sporen und -Hyphen an der Luft getrocknet. Dieses Material wird nachstehend als Inokulum bezeichnet.

In einer weiteren Ausführungsform werden Sporen der erfindungsgemäßen Pilze bereitgestellt. Der Ausdruck Sporen bedeutet Chlamydosporen, d.h. für die arbuskulären Mykorrhizapilze typische Strukturen, die der Vermehrung und Verbreitung der Pilze sowie der Überdauerung der Pilze in Abwesenheit von Wurzelm geeigneter Wirtspflanzen dienen. Die erfindungsgemäßen Sporen sind im üblicher Art und Weise erhältlich, z.B. durch das Verfahren von Esch et al, 1994.

In einer weiteren Ausführungsform werden Rekultivierungsmittel für durch Schwermetalle, Radionuklide, Herbizide und/oder Salze verseuchte Böden bereitgestellt, umfassend isolierte und gereinigte Pilze der Gattung *Glomus*, die mit Metallophyten ein arbuskuläres Mykorrhizageflecht ausgebildet haben. Weiterhin werden Rekultivierungsmittel für durch Schwermetalle, Radionuklide, Herbizide und/oder Salze verseuchte Böden bereitgestellt, umfassend Sporen erhältlich von Pilzen der Gattung *Glomus*, die mit Metallophyten ein arbuskuläres Mykorrhizageflecht ausgebildet haben. Insbesondere bevorzugt sind Rekultivierungsmittel, umfassend die erfindungsgemäß isolierten und gereinigte Pilze der Gattung *Glomus*, die mit Metallophyten der Art *Viola calaminaria* oder *Viola guestphalica* ein arbuskuläres Mykorrhizageflecht ausgebildet haben. Weiterhin insbesondere bevorzugt sind Rekultivierungsmittel, umfassend Sporen erhältlich von isolierten und gereinigte Pilzen der Gattung *Glomus*, die mit Metallophyten der Art *Viola calaminaria* oder *Viola guestphalica* ein arbuskuläres Mykorrhizageflecht ausgebildet haben. Der Ausdruck Rekultivierungsmittel bedeutet, daß die Pilze, Pilzsporen und -hyphen z.B. mit Torf und/oder Sand bzw. Erde ausgebracht werden können. Die Wahl dieser 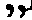 Träger"-Materialien ist hierbei jedoch nicht entscheidend. Ebenfalls gutgeeignet ist das übliche Blähtonverfahren.

In einer weiteren Ausführungsform werden die erfindungsgemäßen Pilz und/oder Sporen zur Rekultivierung verseuchter Böden, insbesondere von mit Schwermetallen, Radionukliden, Herbiziden oder Salzen verseuchten Böden, verwendet. Der Ausdruck Rekultivierung bedeutet z.B. Begrünung mit Pflanzen oder Nutzpflanzen von Haldenstandorten, die bisher nicht mit Pflanzen begrünbar sind. Ferner bedeutet Rekultivierung landwirtschaftliche Nutzbarmachung mit Pflanzen oder Nutzpflanzen von Böden, die durch z.B. Versalzung, Herbizide, Radionuklide verseucht sind. Der Ausdruck Pflanzen oder Nutzpflanzen bedeutet landwirtschaftlich genützte Pflanzen wie Gräser, Klee, Mais, Weizen, Gerste, Soja, Baumwolle, Luzerne, Erdbeeren, Wein, Kaffee, Obsthölzer, z.B. Apfel, Pflaume, Kirsche und Zitrusfrüchte. Dazu werden die erfindungsgemäßen Pilze und/oder Sporen in zur Rekultivierung ausreichender Menge in den verseuchten Boden übertragen. Im Stand der Technik sind verschiedene Verfahren zum Ausbringen von Mykorrhiza-Pilzen und/oder deren Sporen in der Landwirtschaft beschrieben; vgl. Baltruschat, H., Z., (1987); Elmes, R.P. et al., (1983), 437-441; Powell, C.L., (1986). Pflanzen können auch in Vorkultur z.B. im Gewächshaus oder im Freiland inokuliert und erst nach Ausbildung einer Mykorrhiza auf die kontaminierten Böden umgepflanzt werden.

Die Figuren dienen der Erläuterung der Erfindung.

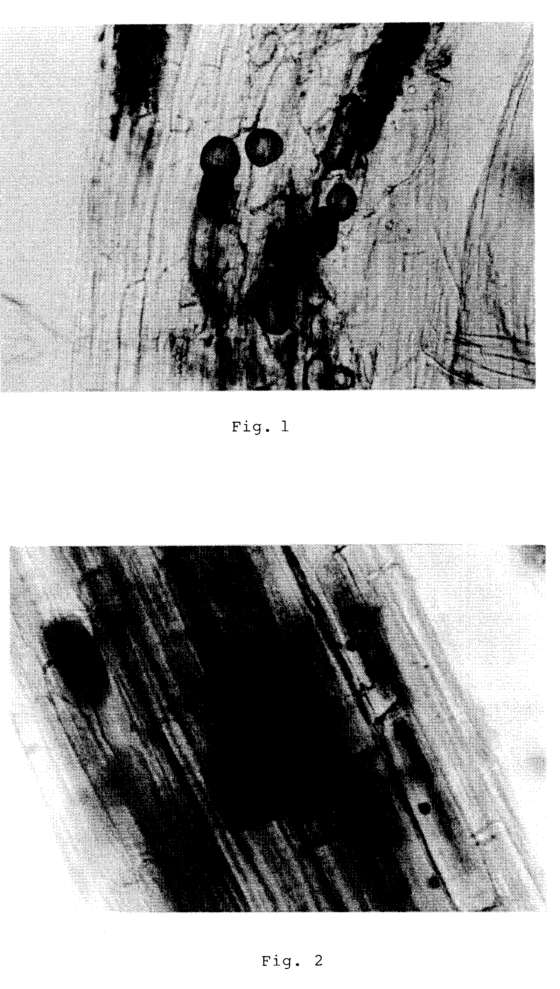

Figur 1 zeigt ein Wurzelpräparat von *Viola calaminaria* mit arbuskulären Mykorrhiza-Pilzen der Gattung *Glomus*.
Figur 2 zeigt ein Wurzelpräparat von *Zea mays* mit arbuskulären Mykorrhiza-Pilzen der Gattung *Glomu*, die von Wurzeln von *Viola calaminaria* isoliert wurden.

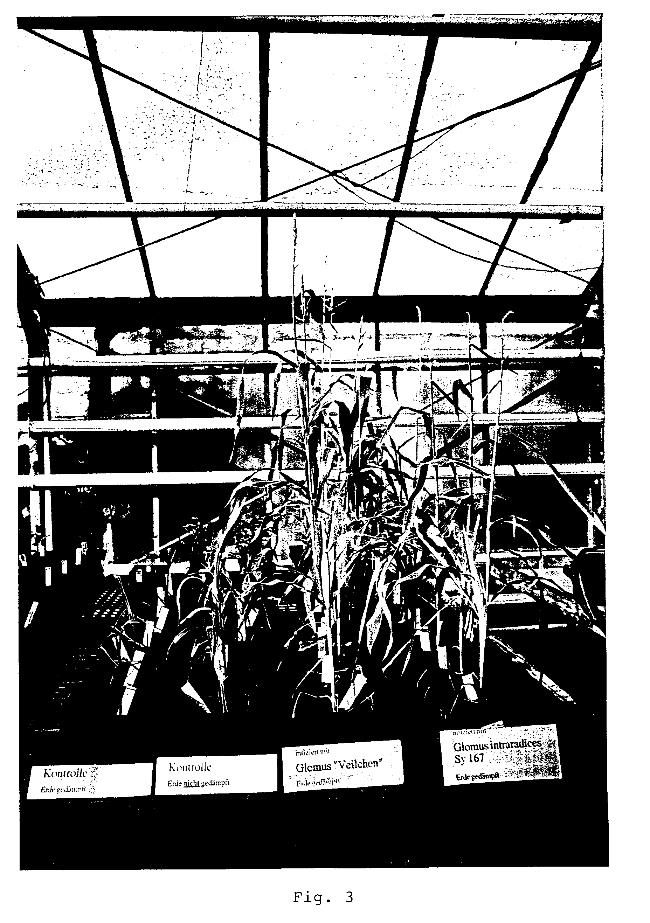

Figur 3 zeigt vier Monate alte *Zea mays*-Pflanzen, die in Schwermetall-haltiger Erde aus Stolberg-Breinigerberg kultiviert wurden. Die Pflanzen wurden in Kontrollerde (gedämpfte Erde), Kontrollerde (ungedämpfte Erde), mit dem erfindungsgemäßen Pilzisolat versetzter Erde und mit einem Kontrollpilzisolat (*Glomus intraradices* Sy167) versetzter Erde kultiviert.

Die nachstehenden Beispiele erläutern die Erfindung und sind nicht als einschränkend aufzufassen.

### Beispiel 1:

### Isolierung, Reinigung und taxonomische Bestimmung von AM-Pilzen der Gattung Glomus

Ca. 80% aller Pflanzen sind potentielle Wirtspflanzen für Arbuskuläre Mykorrhiyapilze der Gattung *Glomus*. Daher sind die Pilze in praktisch allen Böden zu finden, die von Vegetation bedeckt sind. Bevorzugt sind Standorte mit natürlicherweise hohem Schwermetallgehalt, die durch Erzabbau, Verhüttung oder andere anthropogene Einflüsse dauerhaft hohe Schwermetallgehalte im Boden aufweisen. Insbesondere bevorzugt sind Standorte im und am Rande des Naturschutzgebietes "Schlangenberg" in Stolberg-Breiningerberg, an denen Metallophyten der Gattung *Viola calaminaria* gefunden werden. Weiterhin insbesondere Bevorzugt sind Standorte im und am Rande des Naturschutzgebietes 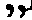 Bleikuhlen" in Lichtenberg-Blankenrode, an denen Metallophyten der Gattun*g Viola guestphalica* gefunden werden.

Die erfindungsgemäßen Pilze wurden folgendermaßen isoliert: Nicht speziell behandelte, mykorrhizierte Wurzeln von *Viola calaminaria* wurden zusammen mit steril gekeimten Maiskörnern in Töpfe mit einer Mischung aus 90% Lecaton / 10 % Einheitserde gelegt. Die aus diesen Wurzelsegmenten von *Viola* auswachsenden *Glomus*-Hyphen haben dann die Maiswurzeln infiziert. Nach 90 Tagen wurde das Substrat einschließlich Maiswurzeln, *Glomus*-Sporen und -Hyphen an der Luft getrocknet.

Die taxonomische Bestimmung der Pilze zur Gattung *Glomus* erfolgte anhand der Sporenmorphologie in üblicher Weise.

### Beispiel 2:

### Isolierung und Reinigung von Sporen des AM-Pilzes der Gattung Glomus

Als Standardmethode zur Isolierung von AM-Sporen gilt das Aussieben der Sporen aus wäßrigen Bodenaufschlämmungen mit anschließender Saccharose-Dichtegradienten-zentrifugation (Werner, 1987). Das hier angewendete Verfahren entspricht der von Esch et al. (1994) publizierten Methode. Etwa 1 l des mit Pilzmaterial infizierten Substrates wurde in 5 l Wasser aufgenommen. Durch kräftiges Rühren wird das Ablösen von Sporen und anderem organischen Material von den Substratpartikeln erreicht. Das Wasser-Substrat-Gemisch wurde anschließend über eine Siebkombination absteigender Porenweite gegeben (1 mm; 0,5 mm; 0,08 mm; 0,45 mm). Je nach Sporengröße des eingesetzten AM-Isolats wurde die 45 µm- oder 80 µm-Fraktion weiter verwendet. Die so erhaltene Sporenfraktion wurde mit Wasser in eine Petrischale gespült. Durch leichtes kreisförmiges Schütteln wurden die Sporen aufgewirbelt, was zu einer groben Abtrennung von Verunreinigungen wie Wurzel- und Sandpartikeln, aber auch Kleininsekten und Nematoden führt. Die aufgewirbelten Sporen wurden mit einer 20 ml-Spritze abgesaugt, an deren Spitze ein Gummischlauch (Innendurchmesser ca. 2 mm) befestigt war. Etwa 40 ml dieses Wasser- Sporengemisches wurden in großen Glaszentrifugenröhrchen mit gleichem Volumen 70% (w/v) Saccharoselösung unterschichtet und 10 min bei 1400g zentrifugiert (Hettich Rotixa KS). Die sich nun in der Saccharose-Wasser-Grenzschicht befindlichen Sporen wurden mit der o.a. Spritze abgenommen und zur Entfernung der Saccharose auf dem 45 µm-Sieb gründlich gewaschen. Zur weiteren Reinigung der Sporenfraktion wurde diese wiederum in eine Petrischale gespült. Über offener Gasflamme auf 100-200 µm Spitzendurchmesser ausgezogene Pasteurpipetten, die an ihrem oberen Ende mit einem Gummischlauch versehen wurden, dienten zum Abpipettieren der Sporen, bzw. der noch vorhandenen Verunreinigungen in entsprechende Gefäße. Für die Aufbewahrung der Sporen bei -20°C erwiesen sich 1 ml -Reaktionsgefäße als gut geeignet.

### Beispiel 3:

### Inokulumproduktion des AM-Pilz der Gattung Glomus

Für die Inokulumproduktion bzw. -vermehrung wurde das Blähtonverfahren verwendet. Hierbei sind die Blähtonpartikel aufgrund ihrer porösen Oberfläche in der Lage, besonders viele Chlamydosporen und Pilzmycel zu binden (Dehne und Backhaus 1986). Um die Gefahr einer Fremdinfektion zu verringern, wurde zur Inokulumvermehrung eine andere Wirtspflanze als die später verwendete Versuchspflanze benutzt (Menge, 1983 und 1986). Zur Inokulumvermehrung wurde *Tagetes* eingesetzt, da diese von den verwendeten *Glomus*- Isolaten schnell infiziert wurde und zudem einer Verbreitung von *Pythium* mit dem Inokulum entgegenwirkt (v. Alten et al., 1990). Die Anzucht der Pflanzen erfolgte in nicht sterilen 1,5-l-Plastiktöpfen, die als Substrat 1,1 l Lecaton^{R} (2-4 mm, Porenvolumen 75%; Leca Deutschland, Halstenbek) und 0,1 l Inokulum enthielten. In jeden Topf wurden drei oberflächensterilisierte Tagetessamen gegeben. Nach einer Wachstumsperiode von zehn bis zwölf Wochen wurde zur Stimulierung der Sporulation der AM-Pilze die Bewässerung der Pflanzen eingestellt. Nach weiteren drei bis vier Wochen wurden die Pflanzen geerntet. Das Inokulum wurde in Plastiktüten bei Raumtemperatur gelagert, wobei das Material seine Infektiosität über mehrere Jahre hinweg behalten soll (Grunewaldt-Stöcker, 1989).

### Beispiel 4:

### Kultivierung von mykorrhiziertem und nicht-mykorrhiziertem Mais und von mykorrhiziertem und nicht-mykorrhiziertem Galmeiveilchen in Schwermetallerden

Es wurde Erde folgender Standorte für die Kultivierung von Mais eingesetzt:
- Erosionsfurche am Rand des Schlangenberges, Stolberg-Breinigerberg (Topographische Karte des Landesvermessungsamtes NRW, L 5302 Aachen, 1:50 000)
- Abraumhalde der Erzabbaufläche Breitenbenden (Bleibergbau) an der Straße zwischen Kalenberg und Kallmuth bei Mechernich / Eifel (Topographische Karte des Landesvermessungsamtes NRW, L 5504 Schleiden, 1:50 000)

Es wurden insgesamt 9 Kulturversuche (4x Mais in 2 verschiedenen Schwermetallerden, 3x Luzerne, 1x Gerste, 1x *Viola guestphalica*) mit prinzipiell gleichen Resultaten zu unterschiedlichen Zeiten mit schwermetallhaltiger und nicht schwermetallhaltiger Erde durchgeführt. Es wurden etwa 75% der jeweils isolierten Bodenmenge von etwa 50-100 kg für 4 Stunden gedämpft, d.h. sterilisiert, (90°C heißer Wasserdampf, kein Überdruck) um die indigene Bodenflora abzutöten. Nach dem Abkühlen auf etwa 25° C wurden die Böden mit 20% Inokulum des jeweiligen Pilzisolates des erfindungsgemäßen Pilzes und/oder der erfindungsgemäßen Sporen, bzw. mit 20% Lecaton (Lecaton®, ⌀ 2-4mm, Porenvolumen 75%, Leca Deutschland, Halstenbek) in den Kontrollansätzen, versetzt. Die oberflächensterilisierten (Sterilisierung mit 70% Ethanol für 5 min., vgl. Neuer et al.) Maiskörner wurden unmittelbar nach dem Ankeimen auf normalen Böden (Mais wurde für drei Tage in sterilen Petrischalen mit angefeuchtetem Filterpapier vorgekeimt und dann in die Töpfe ausgelegt) zu zweit in mit schwermetallhaltigem, mit dem AM-Pilzisolat versetzten Boden gefüllte 1,5 l-Töpfe ausgesät. Die Samen der Galmeiveilchen wurden nicht oberflächensterilisiert, sondern zunächst direkt auf angefeuchtete Einheitserde (Typ ED 73, Patzer KG, Sinntal-Jossa) ausgesät und lichtfrei abgedeckt. Nach etwa 2 Wochen bei 25-30°C erfolgte die Keimung. Etwa 3-4 Wochen nach der Aussaat wurden die Galmeipflänzchen zu zweit in mit Schwermetallerde gefüllte 0,6 l-Töpfe pikiert. Die Hälfte der Pflanzen wurde in sterilisierte Erde umgepflanzt, die mit 20% (v/v) sterilem Lecaton vermischt war. Die anderen Pflanzen wurden in sterilisierte Erde umgepflanzt, die mit 20% (v/v) Inokulum vermischt worden war.

**Tabelle 1**

| Analyse der Schwermetallgehalte in der Bodenlösung. Angabe in mg/l Bodenlösung: | | |
|---|---|---|
| Boden: | Breinigerberg | Mechernich |
| Mn | 0,07 | 1,6 |
| Fe | 0,07 | 0,09 |
| Ni | 0,02 | 0,02 |
| Cu | 0,13 | 0,07 |
| Zn | 1,41 | 0,55 |
| As | 0,045 | 1,01 |
| Hg | 0,14 | >0,01 |
| Pb | 0,02 | 13 |
| Bi | <0,01 | 0,3 |

Die untersuchten mykorrhizierten Pflanzen ( Mais, Gerste, Luzerne) wiesen im Vergleich zu den nicht mykorrhizierten Kontrollpflanzen unter Gewächshausbedingungen teilweise mehr als 10fach erhöhte Sprossgewichte auf. Mit den erfindungsgemäßen Pilzen der Gattung *Glomus* , isoliert von *Viola calaminaria,* inokulierte Pflanzen hatten im Vergleich zu den Pflanzen, die mit dem Kontrollisolat *Glomus intraradices* Sy167 inokuliert waren, ein bis zu doppelt so hohes Gewicht. Die Stärke der beobachteten Wachstumseffekte war hierbei abhängig von den verwendeten Pflanzenarten und den eingesetzten Schwermetallerden.

### Beispiel 5:

### Kulturbedingungen zur Anzucht der Versuchspflanzen

Die Maispflanzen wurden im Gewächshaus bei einer Temperatur von 20-35°C und einer relativen Luftfeuchte von etwa 70% angezogen. Als zusätzliche Lichtquellen zum normalen Tageslicht dienten Hochdruck-Quecksilberdampflampen (HPL-N, 400W, Phillipps) und Hochdruck-Metallhalogendampflampen (HPL-T, 400W, Phillipps), die eine Tageslänge von mindestens 15 Stunden gewährleisteten. Bei künstlicher Beleuchtung lag die Lichtintensität 30 cm oberhalb der Töpfe bei etwa 150 µEm⁻²s⁻¹. Bei starker Sonneneinstrahlung wurden bis zu 600 µEm⁻²s⁻¹ erreicht.

Sowohl hohe Temperaturen von etwa 30-35° C als auch hohe Lichtintensitäten von etwa 200-600 µEm⁻²s⁻¹ stimulierten die Infektion der Pflanzen mit Mykorrhizapilzen, wie auch die Sporenbildung. Die Töpfe mit den Pflanzen wurden auf Gitterroste gestellt, um Staunässe in den Töpfen zu vermeiden, da diese eine erfolgreiche Mykorrhizierung verhindert (Sieverding, 1980; Saif, 1981). Die Pflanzen wurden täglich gegossen und einmal wöchentlich mit einer phosphatfreien HOAGLAND-Lösung gedüngt (Eschrich, 1976) (100ml/Topf). Einmal im Monat erhielten die Maispflanzen Phosphat in Form des leicht verfügbaren KH₂PO_{4.} Je Topf wurde 100 ml Nährlösung der in Tabelle 2 beschriebenen Zusammensetzung verabreicht.

**Tabelle 2**

| Zusammensetzung der Hoagland Nährlösung | | |
|---|---|---|
| **Makroelemente** | **Verbindung** | **Konzentration [µM]** |
| K, N | KNO₃ | 5000 |
| Ca, N | Ca(NO₃)₂ x 4H₂O | 5000 |
| Mg, S | MgSO₄ x 7H₂O | 2000 |
| K, P | K₂HPO₄ | 1000* |

| **Mikroelemente** | | |
|---|---|---|
| Cl | KCl | 50 |
| B | H₃BO₃ | 25 |
| Mn | MnSO₄ x H₂O | 5 |
| Zn | ZnSO₄ x H₂O | 2 |
| Cu | CuSO₄ x H₂O | 0,5 |
| Mo | (NH₄)₆Mo₇O₂₇ x H₂O | 0,014 |
| Fe | Fe-EDTA | 40** |

| | | |
|---|---|---|
| * KH₂PO₄ wurde nur für die phosphathaltige Nährlösung zugegeben; sonst wurden 6000 statt 5000 µM KNO₃ eingesetzt. | | |
| ** Für die Fe-EDTA- Stammlösung wurden 3,72 g Na₂EDTA und 2,78 g FeSO₄ x 7H₂O bei 80°C in1 l H₂O gelöst. | | |

Die Galmeiveilchen (*Viola calaminaria* LEJ.und *Viola guestphalica* NAUENB.) wurden im Gewächshaus bei einer Temperatur von ca. 15-30°C und einer relativen Luftfeuchte von 70% gehalten. Diese Bedingungen entsprachen im wesentlichen denen des Freilands in Frühjahr und Sommer. Eine zusätzliche Belichtung der Pflanzen erfolgte nicht. Die Pflanzen wurden täglich gegossen.

### Beispiel 6:

### Ernte der Versuchspflanzen und Bestimmung des Mykorrhizierungsgrades

Die Ernte der Versuchspflanzen erfolgte beim Mais nach etwa 14 bis 15 Wochen und beim Galmeiveilchen nach etwa 28 Wochen. Die Wurzeln wurden unter fließendem Wasser gründlich gesäubert und anschließend mittels mehrerer Lagen Zellstoff getrocknet. Nach der Ermittlung des Frischgewichts durch Wiegen wurden Wurzelstücke zur Bestimmung des Mykorrhizierungsgrades entnommen. Anschließend erfolgte die Bestimmung des Trockengewichts. Dazu wurde das Wurzel- und Sproßmaterial in Aluminiumtiegeln für 24 Stunden bei 105°C getrocknet.

### Beispiel 6.1:

### Färbung der Wurzeln mit Lactophenolblau

Nach der Säuberung des Wurzelmaterials wurde von jeder Pflanze an verschiedenen Bereichen des Wurzelballens ein Wurzelstück entnommen, in Schnappdeckelgläschen überführt und in 5 ml FAA-Fixierlösung fixiert. Bis zur Färbung wurden die Wurzelproben bei Raumtemperatur im Dunkeln gelagert.

### Zusammensetzung der FAA-Fixierlösung:

- 45,85% H₂O
- 45,85% (v/v) Ethanol
- 6% (v/v) Formaldehyd
- 2,3% (v/v) Eisessig

Zur eindeutigen Identifizierung von Pilzstrukturen im mikroskopischen Bild erfolgte die Färbung der fixierten Wurzelabschnitte mit Lactophenolblau in Abwandlung der üblichen Färbemethoden von Phillips und Hayman (1970) und Kormanik et al. (1980), wie folgt: Zunächst wurden die Wurzeln zur Aufhellung für 35 min bei 90°C im Heizblock mit 10% KOH inkubiert. Nach mehrfachem gründlichem Spülen mit Leitungswasser wurde das Material für 10 min in 3,7% HCl bei Raumtemperatur angesäuert und anschließend durch 90 min Inkubation bei 22°C in Lactophenolblau (C.I. Nr.47780; 1 g/l; pH 2,3; Merck) angefärbt. Zum Entfärben des Pflanzengewebes wurden die Wurzeln in 50% Lactat überführt und bis zur Bonitierung gelagert. Der Ausdruck Bonitierung bedeutet mikroskopische Bestimmung des Mykorrhizierungsgrades.

### Beispiel 6.2:

### Mikroskopische Bestimmung des Mykorrhizierungsgrades

Zur Bonitierung des Mykorrhizierungsgrades wurde die von Schmitz et al. (1991) modifizierte "grid-line-intersect"- Methode nach Ambler und Young (1977) benutzt. Hierzu wurden die mit Lactophenolblau gefärbten Wurzelsegmente auf einem Objektträger ausgelegt und unter dem Mikroskop (Zeiss PM6) bei 125facher Vergrößerung (Durchsicht, Hellfeld) betrachtet. Der so erhaltene Bildauschnitt entspricht einer Länge von 1,4 mm. Jeder innerhalb eines solchen Bildausschnittes sichtbare Wurzelabschnitt wurde auf das Auftreten von Mycel, Arbuskeln und Vesikeln hin überprüft. Dabei gilt ein Segment als mykorrhiziert, wenn mindestens eine der genannten Pilzstrukturen auftritt. Es wurden jeweils etwa 300 Wurzelabschnitte der einzelnen Pflanzen untersucht.
Die Mykorrhizierungsgrade, d.h. prozentualer Anteil der Wurzelsegmente mit Mykorrhiza-Strukturen in einer Stichprobe von 300 Wurzelsegmenten pro Pflanze, lagen in den Kulturversuchen mit Mais und Luzerne zwischen 0 und 5% (nicht inokulierte Pflanzen) bzw. zwischen 50 und 95% (inokulierte Pflanzen).

### Literaturverzeichnis

Ambler,J.R.; Young,J.L. (1977) - Techniques for determining root length infected by vesicular-arbuscular mycorrhizae - Soil Sci. Soc. Am. J. 41, 551-556
Allen,E.B.; Cunningham,G.L. (1983) - Effects of vesicular-arbuscular mycorrizae on Distichlis spicata under three salinity levels - New Phytol. 93, 227-236
Alten, von,H.; Beeck,C.; Schönbeck,F. (1990) - Aspects of infectivity and efficiency of VA-mycorrhiza inoculum on expanded clay, Abstracts: 8th NACOM (North American Conference On Mycorrhizae) - Innovation and hierachical integration - Jackson, Wyoming, September 1990
Baker,A.J.M.; Walker,P.L. (1990) - Ecophysiology of metal uptake by tolerant plants. In: Shaw,A.J., (Ed.): Heavy metal tolerance in plants: evolutionary aspects - CRC-Press, Boca Raton, Florida
Baltruschat, H., Z. PflKrankh. PflSchutz 94, (1987), 419-430
Dehne,H.W.; Backhaus,G.F. (1986) - The use of vesicular-arbuscular mycorrhizal fungi in plant production. I. Inoculum production - J. of Plant Diseases and Protection 93, 415-424
Dueck,T.A.; Visser,P.; Ernst,W.H.O.; Schat,H. (1986) - Vesicular-arbuscular mycorrhizae decrease zinc-toxicity to grasses growing in zinc-polluted soil - Soil Biol. Biochem. 18, 331-333
Elmes, R.P. et al., Ann. Appl. Biol. 104, (1983), 437-441
Ernst,W.H.O. (1972) - Schwermetallresistenz und Mineralstoffhaushalt - Forschungsber. Land Nordrhein-Westfalen 2251, 1-38
Ernst,W.H.O. (1982) - Schwermetallpflanzen - In: Kinzel, Pflanzenökologie und Mineralstoffwechsel, Verlag Eugen Ulmer Stuttgart
Esch,H.; Hundeshagen,B.; Schneider-Poetsch,Hj.; Bothe,H. (1994) - Demonstration of abscisic acid in spores and hyphae of the arbuscular-mycorrhizal fungus Glomus and in the N2-fixing cyanobacterium Anabaena variabilis - Plant Science 99, 9-16
Eschrich,W. (1976) - Strasburger's Kleines Botanisches Praktikum für Anfänger 17. Aufl., Gustav Fischer Verlag, Stuttgart-New York
Estaun,M.V. (1989) - Effect of sodium chloride and mannitol on germination and hyphal growth of the vesicular-arbuscular mycorrhizal fungus Glomus mosseae - Agric. Ecosyst. Environ. 29, 123-129
Gildon,A.; Tinker,P.B. (1981) - A heavy metal-tolerant strain of a mycorrhizal fungus - Trans. Br. Mycol. Soc. 77, 648-649
Gildon,A.; Tinker,P.B. (1983) - Interactions of vesicular-arbuscular mycorrhizal infection and heavy metals in plants. I. The effect of heavy metals on the development of vesicular-arbuscular mycorrhizas - New Phytol. 95, 247-261
Griffioen,W.A.J. (1994) - Characterization of a heavy metal-tolerant endomycorrhizal fungus from the surroundings of a zinc refinery - Mycorrhiza 4, 197-200
Grunewaldt-Stöcker,G. (1989) - Microscopic characterization of VAM-fungal structures on expanded clay for inoculum production - Agric. Ecosystems Environm. 29, 179-182
Harborne,J.B. (1995) - Ökologische Biochemie: Eine Einführung - Spektrum Akad. Verl., Heidelberg, Berlin, Oxford
Hirrel,M.C. (1981) - The effect of sodium and chloride salts on the germination of Gigaspora margarita - Mycologia 73, 610-617
Ho,I. (1987) - Vesicular-arbuscular mycorrhizae of halophytic grasses in the Alvard desert of Oregon - Nothwest Sci. 61, 148-151
Juniper,S.; Abbott,L. (1993) - Vesicular-arbuscular mycorrhizas and soil salinity - Mycorrhiza 4, 45-57
Khan,A.G. (1974) - The occurrence of mycorrhizas in halophytes, hydrophytes and xerophytes, and of Endogone spores in adjacent soils - J. Gen. Microbiol. 81, 7-14
Killham,K.; Firestone,M.K. (1983) - Vesicular-arbuscular mycorrhizal mediation of grass response to acidic and heavy metal deposition - Plant Soil 72, 39-48
Kormanik,P.P.; Bryan,W.C.; Schultz,R.C. (1980) - Procedures and equipment for staining large numbers of plant root samples for endomycorrhizal assay - Can. J. Microbiol. 26, 536-538
Levitt,J. (1980) - Responses of plants to environmental stresses - Academic Press, New York
Menge, J.A. (1983) - Utilization of vesicular- arbuscular mycorrhizal fungi in agriculture - Can. J. Bot. 61, 1015-1024
Menge,J.A. (1986) - Inoculum production, In: VA mycorrhiza, C.L.Powell and D.J. Bagyaraj Eds., CRC Press, Boca Raton, USA, 187-203
Phillips,J.M.; Hayman,D.S. (1970) - Improved procedures for cleaning roots and staining parasitic and vesicular arbuscular mycorrhizal fungi for rapid assessment of infection - Trans. Br. mycol. Soc., 158-168
Pond,E.C.; Menge,J.A.; Jarrell,W.M. (1984) - Improved growth of tomato in salinized soil by vesicular-arbuscular mycorrhizal fungi collected from saline soils - Mycologia 76, 74-84
Powell, C.L., In: VA mycorrhiza, C.L. Powell und D.J. Bagyaraj, Herausgeber, CRC Press, Boca Raton, USA, (1986), 205-222
Rozema,J.; Arp,W.; Van Diggelen,J.; Van Esbroek,M.; Broekman,R.; Punte,H. (1986) - Occurence and ecological significance of vesicular-arbuscular mycorrhiza in the salt marsh environment - Acta Bot. Neerl. 35, 457-467
Saif,S.R. (1981) - The influence of soil aeration on the efficiency of vesicular-arbuscular mycorrizae. I. Effect of soil oxygen on growth and mineral uptake in Eupatorium odoratum L. inoculated with Glomus macrocarpus - New Phytol., 88, 649-659
Schlee,D (1992) - Ökologische Biochemie - Fischer Verlag, Jena
Schmitz,O.; Danneberg,G.; Hundeshagen,B.; Klingner,A; Bothe,H. (1991) - Quantification of vesicular-arbuscular mycorrhiza by biochemical parameters - J. Plant Physiol. 139, 106-114
Sieverding,E. (1980) - Einfluß der Bodenfeuchte auf die Entwicklung und Effektivität der vesikulär-arbuskulären Mykorrhiza - Dissertation, Göttingen
Weissenhorn,I.; Leyval,C.; Berthelin,J. (1993) - Cd-tolerant arbuscular mycorrhizal (AM) fungi from heavy-metal polluted soils - Plant and Soil 157, 247-256
Weissenhorn,I.; Glashoff,A.; Leyval,C.; Berthelin,J. (1994) - Differential tolerance to Cd and Zn of arbuscular mycorrhizal (AM) fungal spores isolated from heavy metal-polluted and unpolluted soils
Weissenhorn,I.; Leyval,C.; Belgy,G.; Berthelin,J. (1995b) - Arbuscular mycorrhizal contribution to heavy metal uptake by maize (Zea mays L.) in pot culture with contaminated soil - Mycorrhiza 5, 245-251
Werner,D. (1987) - Pflanzliche und mikrobielle Symbiosen; Georg Thieme Verlag, Stuttgart/New York

## Patentansprüche

1. Ein isolierter und gereinigter Pilz der Gattung *Glomus*, der mit Metallophyten der Art *Viola calaminaria* oder *Viola guestphalica* ein arbuskuläres Mykorrhizageflecht ausgebildet hat.

2. Sporen erhältlich aus dem Pilz nach Anspruch 1.

3. Rekultivierungsmittel für durch Schwermetalle, Radionuklide, Herbizide und/oder Salze verseuchte Böden, umfassend isolierte und gereinigte Pilze der Gattung *Glomus*, die mit Metallophyten ein arbuskuläres Mykorrhizageflecht ausgebildet haben, in zur Rekultivierung ausreichender Menge.

4. Rekultivierungsmittel für durch Schwermetalle, Radionuklide, Herbizide und/oder Salze verseuchte Böden, umfassend Sporen erhältlich von Pilzen der Gattung *Glomus*, die mit Metallophyten ein arbuskuläres Mykorrhizageflecht ausgebildet haben, in zur Rekultivierung ausreichender Menge.

5. Rekultivierungsmittel für durch Schwermetalle, Radionuklide, Herbizide und/oder Salze verseuchte Böden, umfassend isolierte und gereinigte Pilze und/oder deren Sporen nach Anspruch 1 oder 2, in zur Rekultivierung ausreichender Menge.

6. Rekultivierungsmittel nach einem der Ansprüche 3 bis 5 für durch Schwermetalle verseuchte Böden.

7. Verwendung von isolierten und gereinigten Pilzen der Gattung *Glomus*, die mit Metallophyten ein arbuskuläres Mykorrhizageflecht ausgebildet haben, zur Rekultivierung von durch Schwermetalle, Radionuklide, Herbizide und/oder Salze verseuchten Böden, in zur Rekultivierung ausreichender Menge.

8. Verwendung von Sporen, erhältlich von Pilzen der Gattung *Glomus*, die mit Metallophyten ein arbuskuläres Mykorrhizageflecht ausgebildet haben, zur Rekultivierung von durch Schwermetalle, Radionuklide, Herbizide und/oder Salze verseuchten Böden, in zur Rekultivierung ausreichender Menge.

9. Verwendung der Pilze und/oder deren Sporen nach Anspruch 1 oder 2 zur Rekultivierung von durch Schwermetalle, Radionuklide, Herbizide und/oder Salze verseuchten Böden, in zur Rekultivierung ausreichender Menge.

10. Verwendung nach einem der Ansprüche 7 bis 9 zur Rekultivierung von durch Schwermetalle verseuchten Böden, in zur Rekultivierung ausreichender Menge.
